# EUROPEAN PATENT APPLICATION

(11) **EP 4 509 743 A1**
(43) Date of publication of application: **19.02.2025**
(21) Application number: 23826187.9
(22) Date of filing: 12.06.2023
(51) Int. Cl.: F16K 31/06, A61B 5/0235, A61B 5/022

(54) **SOLENOID VALVE AND APPARATUS**

(30) Priority: 20.06.2022 CN 202210700230
(71) Applicant: Huawei Technologies Co., Ltd., Shenzhen, Guangdong 518129 (CN)
(72) Inventor: WANG, Youhua, Shenzhen, Guangdong 518129 (CN); JIN, Junye, Shenzhen, Guangdong 518129 (CN); ZHANG, Kai, Shenzhen, Guangdong 518129 (CN); XU, Tao, Shenzhen, Guangdong 518129 (CN); WANG, Xin, Shenzhen, Guangdong 518129 (CN)
(74) Representative: MERH-IP Matias Erny Reichl Hoffmann Patentanwälte PartG mbB
(86) International application number: PCT/CN2023/099611
(87) International publication number: WO 2023/246538

(57) **Abstract**

This application relates to the valve field, and in particular, to a solenoid valve and a device. The solenoid valve includes: a valve body, where the valve body has an accommodating cavity; a magnet yoke, where the magnet yoke is disposed in the accommodating cavity, and the magnet yoke has a mounting groove; a coil winding, where the coil winding is disposed in the mounting groove; a valve plug assembly, where the valve plug assembly is disposed in the accommodating cavity, and the valve plug assembly includes a movable coil winding; and a multistable element, where an outer circumferential end of the multistable element is connected to the valve body, an inner circumferential end of the multistable element is connected to the valve plug assembly, and at least one steady-state position is disposed on the multistable element to position the valve plug assembly. When the coil winding is powered on, the magnet yoke can attract the valve plug assembly, and the valve plug assembly drives the multistable element to deform to the steady-state position, so that the valve plug assembly blocks or opens an air path of the solenoid valve. When the valve plug assembly moves, the movable coil winding can generate a magnetic field, and the magnetic field generated by the movable coil can interact with a magnetic field generated by the coil winding, to detect a signal change of the coil winding.

## Description

This application claims priority to Chinese Patent Application No. 202210700230.3, filed with the China National Intellectual Property Administration on June 20, 2022 and entitled "SOLENOID VALVE AND DEVICE", which is incorporated herein by reference in its entirety.

### TECHNICAL FIELD

This application relates to the technical field, and in particular, to a solenoid valve and a device.

### BACKGROUND

A blood pressure status is closely related to human health, and is a key indicator for evaluating a plurality of diseases. With improvement of living standards, people require that a wearable blood pressure meter or a wearable blood pressure gauge can be used anytime and anywhere to dynamically measure blood pressure. When an air pump of the wearable blood pressure gauge is faulty and cannot discharge air, it is required that an airbag can discharge air through an air valve. However, an existing air valve cannot quickly respond, and needs to be always powered on in an inflation process of the airbag, resulting in high energy consumption.

### SUMMARY

This application provides a solenoid valve and a device, to quickly switch the solenoid valve to a normally open state or a normally closed state, to shorten a power-on time of the solenoid valve, thereby reducing energy consumption.

A first aspect of this application provides a solenoid valve, where the solenoid valve includes:
a valve body, where the valve body has an accommodating cavity;
a magnet yoke, where the magnet yoke is disposed in the accommodating cavity, and the magnet yoke has a mounting groove;
a coil winding, where the coil winding is disposed in the mounting groove;
a valve plug assembly, where the valve plug assembly is disposed in the accommodating cavity and can move relative to the valve body, and the valve plug assembly includes a movable coil winding; and
a multistable element, where an outer circumferential end of the multistable element is connected to the valve body, an inner circumferential end of the multistable element is connected to the valve plug assembly, and at least one steady-state position is disposed on the multistable element to position the valve plug assembly.

When the coil winding is powered on, the magnet yoke can drive the valve plug assembly to move, and the valve plug assembly drives the multistable element to deform to the steady-state position, so that the valve plug assembly blocks or opens an air path of the solenoid valve.

When the valve plug assembly moves, the movable coil winding can generate a magnetic field, and the magnetic field generated by the movable coil can interact with a magnetic field generated by the coil winding, to detect a signal change of the coil winding.

In this application, when the coil winding is powered on, a magnetic field is generated, and the magnetic field polarizes the magnet yoke, so that the magnet yoke generates magnetic force, the magnet yoke can attract or repel the valve plug assembly to move, and the valve plug assembly drives the multistable element to move. The outer circumferential end of the multistable element is fastened to the valve body. Therefore, when the multistable element is driven by the valve plug assembly to move, the outer circumferential end of the multistable element is constrained by the valve body, thereby implementing buckling deformation, to reach the steady-state position. After the multistable element reaches the steady-state position, the valve plug assembly can be positioned, that is, movement of the valve plug assembly is limited, so that the valve plug assembly remains in a state of blocking the air path of the solenoid valve, or the valve plug assembly remains in a state of opening the air path of the solenoid valve. In this embodiment, the valve plug assembly is electromagnetically controlled to move to open or block the air path, so that the solenoid valve quickly responds, to discharge air in a timely manner. The multistable element is disposed to limit a position of a valve plug after movement, so that the coil winding can change an air path connectivity status after working (being powered on) for only a short time, thereby reducing energy consumption.

After the coil winding is powered on, a magnetic field is generated, so that the magnet yoke generates magnetic force to drive the valve plug assembly to move. The valve plug assembly drives the movable coil winding to move, so that the movable coil winding generates a magnetic field. The magnetic field generated by the movable coil winding interacts with the magnetic field generated by the coil winding, and affects a signal of the coil winding. Therefore, whether the valve plug assembly normally moves is determined by detecting a signal change of the coil winding, and whether the solenoid valve normally works is determined.

In a possible design, before the multistable element is mounted on the valve body, an upward buckling deformation distance of the multistable element is Y1, and a downward buckling deformation distance of the multistable element is Y2.

After the multistable element is mounted on the valve body, an upward buckling deformation distance of the multistable element is Y3, and a downward buckling deformation distance of the multistable element is Y4.

Y3 < Y1 and Y4 < Y2 are met, so that the multistable element positions the valve plug assembly.

In this application, the valve body limits a buckling deformation amount after the multistable element is mounted on the valve body to be less than a buckling deformation amount before the multistable element is mounted on the valve body, so that the multistable element of the valve body can still have a deformation trend after being mounted, limit movement of the valve plug assembly, and position the valve plug assembly in the state of blocking the air path or in the state of opening the air path.

In a possible design, the multistable element is divided into a plurality of parts along a circumferential direction of the multistable element, and there is an interval between parts of the multistable element.

The valve plug assembly includes a spacer, the spacer is located in the interval, and there is a gap between the adjacent spacer and the multistable element.

When the coil winding is powered on, the magnet yoke can attract the spacer to move.

In this application, after the coil winding is powered on, a magnetic field is generated, and the magnetic field attracts the spacer. The spacer needs to be disposed as close as possible to the coil winding. Therefore, the multistable element is divided into the plurality of parts, and the spacer is disposed in the interval between parts of the multistable element, so that the multistable element is not a block between the spacer and the coil winding, and the spacer can be closer to the coil winding, thereby ensuring that the spacer can be attracted to move.

In a possible design, the magnet yoke includes a first magnet yoke and a second magnet yoke. Along a height direction of the solenoid valve, the first magnet yoke is disposed on one side of the valve plug assembly, and the second magnet yoke is disposed on the other side of the valve plug assembly.

The first magnet yoke has a first mounting groove, and the second magnet yoke has a second mounting groove.

The coil winding includes a first coil winding and a second coil winding, the first coil winding is disposed in the first mounting groove, and the second coil winding is disposed in the second mounting groove.

When the first coil winding is powered on, the first magnet yoke can attract the spacer, and the spacer drives the multistable element to deform to a first steady-state position.

When the second coil winding is powered on, the second magnet yoke can attract the spacer, and the spacer drives the multistable element to deform to a second steady-state position.

In a possible design, the valve body further has a first air intake vent and a first air exhaust vent.

The first magnet yoke further has a first breather hole, the first breather hole communicates with the first air intake vent, the second magnet yoke further has a second breather hole, and the second breather hole communicates with the first air exhaust vent.

The valve plug assembly includes a first sealing element, and the first sealing element is disposed close to the first breather hole.

When the first coil winding is powered on, the first magnet yoke can attract the spacer, and the spacer drives the multistable element to deform to the first steady-state position, so that the first sealing element seals the first breather hole to block the air path.

When the second coil winding is powered on, the second magnet yoke can attract the spacer, and the spacer drives the multistable element to deform to the second steady-state position, so that the first sealing element is away from the first breather hole, the spacer abuts against the second magnet yoke, and the first air intake vent communicates with the first air exhaust vent to open the air path.

In this application, the first breather hole, the second breather hole, and the accommodating cavity communicate with each other, and the first air intake vent and the first air exhaust vent are disposed opposite to each other along the height direction of the solenoid valve.

When the first coil winding is powered on, the second coil winding is in a power-off state, the first coil winding generates a magnetic field, and the magnetic field polarizes the first magnet yoke to generate magnetic force, so that the first magnet yoke can attract the spacer to move in a direction close to the first magnet yoke, the spacer drives the first sealing element to move, and the first sealing element moves to a position at which the first breather hole is blocked. In addition, the spacer drives the multistable element to deform to the first steady-state position, so that the multistable element presses the first sealing element to always maintain a state of blocking the first breather hole, thereby closing the air path of the solenoid valve.

When the second coil winding is powered on, the first coil winding is in a power-off state, the second coil winding generates a magnetic field, and the magnetic field polarizes the second magnet yoke to generate magnetic force, so that the second magnet yoke can attract the spacer to move in a direction close to the second magnet yoke, and the spacer drives the first sealing element to move away from the first breather hole. When the spacer moves to abut against the second magnet yoke, the spacer drives the multistable element to deform to the second steady-state position, so that the multistable element presses the spacer to maintain a state of abutting against the second magnet yoke, and the first sealing element maintains a state of being away from the first breather hole. The first breather hole, the accommodating cavity, and the second breather hole communicate with each other, so that the first air intake vent communicates with the first air exhaust vent, thereby opening the air path of the circuit valve.

Along the height direction of the solenoid valve, a projection surface of the first sealing element can cover a projection surface of the first breather hole, so that the first sealing element can block the first breather hole.

In a possible design, the valve body further has a first air intake vent, a second air intake vent, and a first air exhaust vent.

The first magnet yoke further has a first breather hole, the first breather hole communicates with the first air intake vent, the second magnet yoke further has a second breather hole, and the second breather hole communicates with the second air intake vent.

The valve plug assembly further includes a first sealing element and a second sealing element, the first sealing element is disposed close to the first breather hole, and the second sealing element is disposed close to the second breather hole.

When the first coil winding is powered on, the first magnet yoke can attract the spacer, and the spacer drives the multistable element to deform to the first steady-state position, so that the first sealing element seals the first breather hole, and the second air intake vent communicates with the first air exhaust vent.

When the second coil winding is powered on, the second magnet yoke can attract the spacer, and the spacer drives the multistable element to deform to the second steady-state position, so that the second sealing element seals the second breather hole, and the first air intake vent communicates with the first air exhaust vent.

In this application, along the height direction of the solenoid valve, the first air intake vent and the second air intake vent are disposed opposite to each other, and the first air exhaust vent is disposed on one side of the first air intake vent and the second air intake vent.

When the first coil winding is powered on, the second coil winding is in a power-off state, the first coil winding generates a magnetic field, and the magnetic field polarizes the first magnet yoke to generate magnetic force, so that the first magnet yoke can attract the spacer to move in a direction close to the first magnet yoke, the spacer drives the first sealing element to move, and the first sealing element moves to a position at which the first breather hole is blocked, to block the first air intake vent. In addition, the spacer drives the multistable element to deform to the first steady-state position, so that the multistable element presses the first sealing element to always maintain a state of blocking the first air intake vent, thereby implementing communication between the second air intake vent and the first air exhaust vent.

When the second coil winding is powered on, the first coil winding is in a power-off state, the second coil winding generates a magnetic field, and the magnetic field polarizes the second magnet yoke to generate magnetic force, so that the second magnet yoke can attract the spacer to move in a direction close to the second magnet yoke, the spacer drives the second sealing element to move, and the second sealing element moves to a position at which the second breather hole is blocked, to block the second air intake vent. In addition, the spacer drives the multistable element to deform to the second steady-state position, so that the multistable element presses the second sealing element to always maintain a state of blocking the second air intake vent, thereby implementing communication between the first air intake vent and the first air exhaust vent.

In a possible design, the valve plug assembly further includes a movable valve plug, and the movable coil winding is disposed around an outer ring of the movable valve plug.

When the first magnet yoke attracts the movable valve plug to move, the movable valve plug drives the movable coil winding to move in a direction close to the first coil winding, so that a magnetic field generated by the movable coil winding interacts with a magnetic field generated by the first coil winding, to detect a signal change of the first coil winding, and determine whether the solenoid valve normally works.

When the second magnet yoke attracts the movable valve plug to move, the movable valve plug drives the movable coil winding to move in a direction close to the second coil winding, so that a magnetic field generated by the movable coil winding interacts with a magnetic field generated by the second coil winding, to detect a signal change of the second coil winding, and determine whether the solenoid valve normally works.

In this application, the movable valve plug and the movable coil winding are disposed in a middle region of the spacer and the multistable element, the first sealing element is disposed at one end of the movable valve plug, and a material of the movable valve plug may be iron.

The movable coil winding is a closed circuit. When the movable coil winding is driven to move, a magnetic induction line is cut to generate an induced current, and the induced current generates an induced magnetic field.

When the first coil winding is powered on, the first coil winding generates a magnetic field, so that the first magnet yoke generates magnetic force and can attract the movable valve plug to move towards the first magnet yoke. The movable valve plug drives the movable coil winding to move in a direction close to the first coil winding. A magnetic field generated by the movable coil winding in a moving process interacts with a magnetic field generated by the first coil winding, and affects an electrical signal of the first coil winding. Therefore, a detecting element is used to detect a change of the electrical signal (for example, a current, a voltage, or power) of the first coil winding, to determine whether the first sealing element moves in a direction close to the first breather hole, to determine whether the solenoid valve normally works.

When the second coil winding is powered on, the second coil winding generates a magnetic field, so that the second magnet yoke generates magnetic force and can attract the movable valve plug to move towards the second magnet yoke. The movable valve plug drives the movable coil winding to move in a direction close to the second coil winding. A magnetic field generated by the movable coil winding in a moving process interacts with a magnetic field generated by the second coil winding, and affects an electrical signal of the second coil winding. Therefore, the detecting element is used to detect a change of the electrical signal (for example, a current, a voltage, or power) of the second coil winding, to determine whether the first sealing element moves in a direction away from the first breather hole, to determine whether the solenoid valve normally works.

In a possible design, the valve plug assembly further includes a movable valve plug, and the movable coil winding is disposed around an outer ring of the movable valve plug.

When the first coil winding is powered on and the first magnet yoke attracts the movable valve plug to move, the second coil winding can generate a magnetic field, to detect a signal change of the second coil winding, and determine whether the first coil winding normally works.

When the second coil winding is powered on and the second magnet yoke attracts the movable valve plug to move, the first coil winding can generate a magnetic field, to detect a signal change of the first coil winding, and determine whether the second coil winding normally works.

In this application, when the first coil winding is powered on, a magnetic field is generated. As a result, the second coil winding also generates a magnetic field, the magnetic field generates an electrical signal, and the detecting element is used to detect a change of the electrical signal of the second coil winding. In addition, a magnetic field generated by the movable coil winding when moving also causes a change of the electrical signal of the second coil. The detecting element is used to detect the change of the electrical signal of the second coil winding, to determine whether the first coil winding normally works, and determine whether the first sealing element moves in a direction close to the first breather hole, to determine whether the solenoid valve normally works.

When the second coil winding is powered on, a magnetic field is generated. As a result, the first coil winding also generates a magnetic field, the magnetic field generates an electrical signal, and the detecting element is used to detect a change of the electrical signal of the first coil winding. In addition, a magnetic field generated by the movable coil winding when moving also causes a change of the electrical signal of the first coil. The detecting element is used to detect the change of the electrical signal of the first coil winding, to determine whether the second coil winding normally works, and determine whether the first sealing element moves in a direction away from the first breather hole, to determine whether the solenoid valve normally works.

In a possible design, the mounting groove is an annular groove, and the annular groove is provided with an inner wall and an outer wall.

A tooth groove is disposed on an end face of one end that is of the inner wall and that is close to the spacer.

In this application, the tooth groove on the end face can circulate airflow and increase an area relative to the spacer.

In a possible design, the mounting groove is an annular groove, and the annular groove is provided with an inner wall and an outer wall.

A tooth groove is disposed on an end face of one end that is of the outer wall and that is close to the spacer.

In this application, the tooth groove on the end face can circulate airflow and increase an area relative to the spacer.

In a possible design, the mounting groove is an annular groove, and the annular groove is provided with an inner wall and an outer wall.

Tooth grooves are disposed on end faces of ends that are of the inner wall and the outer wall and that are close to the spacer.

In this application, the tooth groove on the end face can circulate airflow and increase an area relative to the spacer.

In a possible design, the valve body includes a first air intake vent, a first air exhaust vent, and a second air exhaust vent.

The valve plug assembly includes a first sealing element, and the first sealing element is connected to the multistable element.

When the coil winding is powered on, the magnet yoke can attract the spacer, and the spacer drives the multistable element to move and deform, so that the spacer abuts against the magnet yoke, and the first air intake vent, the first air exhaust vent, and the second air exhaust vent communicate with each other.

When the coil winding is powered off, the multistable element deforms and rebounds to a second steady-state position, and drives the first sealing element to seal the first air intake vent.

When the coil winding is powered on, a buckling deformation distance of the multistable element is less than Y4.

In this application, the first air intake vent penetrates a side wall of the valve body along the height direction of the solenoid valve, the first air exhaust vent and the second air exhaust vent are respectively disposed at two ends of the first air intake vent, and the first air exhaust vent and the second air exhaust vent are disposed opposite to each other.

In this embodiment, compared with the first embodiment, one coil winding and one magnet yoke are disposed, and no movable coil winding is disposed.

When the coil winding is not powered on, the multistable element is at the second steady-state position, and the multistable element presses the first sealing element to seal the first air intake vent, thereby closing the air path of the solenoid valve.

When the coil winding is powered on, the coil winding generates a magnetic field, and the magnet yoke is magnetized to generate magnetic force, so that the magnet yoke attracts the spacer to move in a direction close to the magnet yoke. The spacer drives the first sealing element to move away from the first air intake vent, and the spacer moves to abut against the magnet yoke, so that the first air intake vent communicates with the first air exhaust vent and the second air exhaust vent, thereby opening the solenoid valve. In addition, the spacer drives the multistable element to deform, and in this case, a deformation distance of the multistable element is less than a distance of deformation of the multistable element to the second steady-state position.

When the coil winding is powered off, the magnetic field disappears. Because the deformation distance of the multistable element when the coil winding is powered on is less than the distance of deformation of the multistable element to the second steady-state position, in this case, the multistable element deforms and rebounds towards the second steady-state position until the first sealing element reaches the top of the valve body to block the first air intake vent, thereby closing the solenoid valve.

In a possible design, the valve body includes a first air intake vent and a first air exhaust vent.

A first breather hole is disposed on the magnet yoke, and the first breather hole communicates with the first air exhaust vent.

The valve plug assembly further includes a first sealing element and a magnetic element, the first sealing element is disposed at one end that is of the magnetic element and that is close to the first air intake vent, and the magnetic element can magnetize the spacer.

When the coil winding is powered off, the magnet yoke attracts the spacer, and the spacer drives the multistable element to move and deform to a first steady-state position, so that the spacer abuts against the magnet yoke, and the first air intake vent communicates with the first air exhaust vent through the first breather hole, to open the air path.

When the coil winding is powered on, under an action of a magnetic field, the magnet yoke repels the spacer, and the spacer drives the first sealing element to seal the first air intake vent, to block the air path.

In this application, the first air intake vent and the first air exhaust vent are disposed opposite to each other along the height direction of the solenoid valve.

In this embodiment, compared with the first embodiment, one coil winding and one magnet yoke are disposed, no movable coil winding is disposed, the movable valve plug is replaced with the magnetic element, and the magnetic element is a permanent magnet.

When the coil winding is powered off, the magnetic element magnetizes the spacer, and the magnet yoke attracts the spacer to move in a direction close to the magnet yoke, so that the spacer is finally attached to the magnet yoke. The spacer drives the first sealing element to move away from the first air intake vent, and the first air intake vent communicates with the first air exhaust vent, thereby opening the air path of the solenoid valve. In addition, the spacer drives the multistable element to deform to the first steady-state position, and the multistable element is in a stretched state.

When the coil winding is powered on, the coil winding generates a magnetic field. The magnetic field generated by the coil winding is opposite to a magnetic field of the magnetic element, and the spacer and the magnetic element are repelled to move in a direction away from the magnet yoke. Rebound force of the multistable element and repelling force generated by the coil winding press the first sealing element onto the valve body through the spacer, and the first air intake vent is blocked, thereby closing the air path of the solenoid valve.

**In** a possible design, the valve body includes a first air intake vent and a first air exhaust vent.

A first breather hole is disposed on the magnet yoke, and the first breather hole communicates with the first air exhaust vent.

The valve plug assembly further includes a first sealing element and a magnetic element, the first sealing element is disposed at one end that is of the magnetic element and that is close to the first air exhaust vent, and the magnetic element can magnetize the spacer.

When the coil winding is powered off, the magnet yoke attracts the spacer, the spacer drives the sealing element to seal the first breather hole, and the multistable element moves and deforms to a second steady-state position, to block the air path.

When the coil winding is powered on, under an action of a magnetic field, the magnet yoke repels the spacer, and the spacer drives the multistable element to deform, so that the first sealing element is away from the first breather hole, and the first air intake vent communicates with the first air exhaust vent through the first breather hole, to open the air path.

In this application, the first air intake vent and the first air exhaust vent are disposed opposite to each other along the height direction of the solenoid valve.

In this embodiment, compared with the first embodiment, one coil winding and one magnet yoke are disposed, no movable coil winding is disposed, the movable valve plug is replaced with the magnetic element, and the magnetic element is a permanent magnet.

When the coil winding is powered off, the magnetic element magnetizes the spacer, and the magnet yoke attracts the spacer to move in a direction close to the magnet yoke, so that the spacer is finally attached to the magnet yoke. The spacer drives the first sealing element to block the first breather hole, to block the first air exhaust vent, thereby closing the air path of the solenoid valve. In addition, the spacer drives the multistable element to deform to the second steady-state position, and the multistable element is in a stretched state.

When the coil winding is powered on, the coil winding generates a magnetic field. The magnetic field generated by the coil winding is opposite to a magnetic field of the magnetic element, and the spacer and the magnetic element are repelled to move in a direction away from the magnet yoke. Rebound force of the multistable element and repelling force generated by the coil winding drive the first sealing element through the spacer to move in a direction away from the first breather hole, so that the first air intake vent communicates with the first air exhaust vent through the first breather hole, thereby opening the air path of the solenoid valve.

In a possible design, along a height direction of the solenoid valve, a cross-sectional area of the first air intake vent is greater than a cross-sectional area of the magnetic element.

In this application, when the magnetic element moves to be close to the first air intake vent, the first air intake vent is not blocked, thereby ensuring communication between the first air intake vent and the first air exhaust vent.

A second aspect of this application provides a solenoid valve, where the solenoid valve includes:
a valve body, where the valve body has an accommodating cavity;
a first magnet yoke, where the first magnet yoke is disposed in the accommodating cavity, and the first magnet yoke has a first mounting groove;
a first coil winding, where the first coil winding is disposed in the first mounting groove;
a valve plug assembly, where the valve plug assembly is disposed in the accommodating cavity and can move relative to the valve body; and
an elastic element, where the elastic element is disposed in the accommodating cavity, and two ends of the elastic element are respectively connected to the valve plug assembly and an inner wall of the valve body.

Under elastic force of the elastic element, the elastic element can press the valve plug assembly, so that the valve plug assembly blocks or opens an air path of the solenoid valve.

When the first coil winding is powered on, the first magnet yoke can drive the valve plug assembly to move, so that the valve plug assembly can overcome the elastic force of the elastic element to open or block the air path of the solenoid valve.

In this application, the elastic element is disposed to press the valve plug assembly, so that the valve plug assembly may remain in a state of blocking or opening the air path. To change an air path connectivity status, the first coil winding needs to work for only a short time, so that energy consumption is low.

In a possible design, the valve plug assembly includes a spacer and a first sealing element, and the spacer is connected to the first sealing element.

The valve body has a first air intake vent and a first air exhaust vent, and the first sealing element is disposed close to the first air intake vent.

When the first coil winding is powered off, under elastic force of the elastic element, the spacer presses the first sealing element to seal the first air intake vent, to block the air path.

When the first coil winding is powered on, the first magnet yoke attracts the spacer, the spacer overcomes the elastic force of the elastic element to drive the first sealing element to move in a direction away from the first air intake vent, and the first air intake vent communicates with the first air exhaust vent, to open the air path.

In this application, the first air intake vent penetrates a side wall of the valve body along a height direction of the solenoid valve, and the first air exhaust vent is disposed on one side of the first air intake vent along a length direction of the solenoid valve. The elastic element may be a spring. One end of the spring is connected to the spacer, the other end is connected to an inner bottom wall of the valve body, and the spring is mounted in the accommodating cavity in a pre-compressed state.

When the first coil winding is powered off, the elastic element can press the spacer through elastic force of the elastic element, and the spacer abuts against an inner side wall of the valve body, so that the spacer presses the first sealing element to block the first air intake vent, and the air path of the solenoid valve remains in a closed state.

When the first coil winding is powered on, a magnetic field is generated, and the first magnet yoke is magnetized to generate magnetic force. The first magnet yoke attracts the spacer to overcome the elastic force of the elastic element and move in a direction close to the first magnet yoke. The spacer drives the first sealing element to move away from the first air intake vent, so that the first air intake vent communicates with the first air exhaust vent, thereby opening the air path of the solenoid valve.

In a possible design, a recessed portion is disposed on the first magnet yoke, a protrusion portion corresponding to the recessed portion is disposed on the spacer, and the protrusion portion is disposed in the recessed portion.

Along a height direction of the solenoid valve, the protrusion portion can move along the recessed portion.

In this application, in a moving process of the spacer, the protrusion portion can move in the recessed portion. By disposing the protrusion portion and the recessed portion, a contact area between the spacer and the first magnet yoke is increased, thereby enhancing magnetic attraction force.

In a possible design, the valve plug assembly includes a spacer and a first sealing element, and the spacer is connected to the first sealing element.

The valve body has a first air intake vent and a first air exhaust vent, and the first sealing element is disposed close to the first air exhaust vent.

A first breather hole is disposed on the first magnet yoke, and the first breather hole communicates with the first air exhaust vent.

When the first coil winding is powered off, the spacer is pressed under elastic force of the elastic element, so that the first sealing element is away from the first breather hole, and the first air intake vent communicates with the first air exhaust vent through the first breather hole, to open the air path.

When the first coil winding is powered on, the first magnet yoke attracts the spacer, and the spacer overcomes the elastic force of the elastic element to drive the first sealing element to seal the first breather hole, to block the air path.

In this application, the first air intake vent and the first air exhaust vent are disposed opposite to each other along the height direction of the solenoid valve. The elastic element may be a spring. One end of the spring is connected to the spacer, the other end is connected to an inner bottom wall of the valve body, and the spring is mounted in the accommodating cavity in a pre-compressed state.

When the first coil winding is powered off, the elastic element can press the spacer through elastic force of the elastic element, so that the spacer abuts against the valve body. In this case, the first sealing element is away from the first breather hole, and the air path of the solenoid valve remains in an open state.

When the first coil winding is powered on, a magnetic field is generated, and the first magnet yoke is magnetized to generate magnetic force. The first magnet yoke attracts the spacer to overcome the elastic force of the elastic element and move in a direction close to the first magnet yoke. The spacer drives the first sealing element to block the first breather hole, thereby closing the air path of the solenoid valve.

A third aspect of this application provides a device, configured to measure blood pressure, where the device includes:
a device body;
an airbag, mounted on the device body, where the airbag is configured to be worn on a measured part;
an air pump, mounted on the device body, where the air pump is configured to provide fluid to the airbag;
a pressure sensor, mounted on the device body, where the pressure sensor is configured to detect pressure of the airbag;
a solenoid valve, where the solenoid valve is mounted on the device body, and the solenoid valve is the solenoid valve described above; and
a detecting element, where the detecting element is configured to detect whether the solenoid valve normally works.

In this application, the airbag may be a cuff worn on a measured part such as a wrist or an upper wall. The air pump communicates with the airbag, so that the air pump can provide fluid to the airbag and pressurize the airbag, and the fluid is air. The pressure sensor is configured to detect pressure of the airbag. The solenoid valve communicates with the airbag and the air pump, so that when the air pump is faulty and cannot discharge air from the airbag, the airbag can discharge air through the solenoid valve, so that the device normally runs. To feed back a working status of the solenoid valve in real time, the device further includes the detecting element. The detecting element is electrically connected to the solenoid valve to detect whether the solenoid valve normally works, to locate a fault location of the device.

It should be understood that the foregoing general descriptions and the following detailed descriptions are merely used as an example, and should not limit this application.

### BRIEF DESCRIPTION OF DRAWINGS

FIG. 1 is a diagram of a solenoid valve in a closed state according to a first embodiment of this application;
FIG. 2 is a diagram of a structure of a valve plug assembly in FIG. 1;
FIG. 3 is a diagram of the solenoid valve in an open state according to the first embodiment of this application;
FIG. 4 is a diagram of a solenoid valve in which a second air intake vent communicates with a first air exhaust vent according to a second embodiment of this application;
FIG. 5 is a diagram of the solenoid valve in which a first air intake vent communicates with the first air exhaust vent according to the second embodiment of this application;
FIG. 6 is a diagram of a solenoid valve in a closed state according to a third embodiment of this application;
FIG. 7 is a diagram of the solenoid valve in an open state according to the third embodiment of this application;
FIG. 8 is a diagram of a solenoid valve in an open state according to a fourth embodiment of this application;
FIG. 9 is a diagram of the solenoid valve in a closed state according to the fourth embodiment of this application;
FIG. 10 is a diagram of a solenoid valve in a closed state according to a fifth embodiment of this application;
FIG. 11 is a diagram of the solenoid valve in an open state according to the fifth embodiment of this application;
FIG. 12 is a diagram of a connection between a multistable element and a spacer according to this application;
FIG. 13 is a diagram of a connection between a multistable element and a spacer according to this application;
FIG. 14 is a diagram of a structure of a first/second magnet yoke according to this application;
FIG. 15 is a diagram of a structure of a first/second magnet yoke according to this application;
FIG. 16 is a diagram of a structure of a first/second magnet yoke according to this application;
FIG. 17 is a diagram of a status of a multistable element before being processed according to this application;
FIG. 18 and FIG. 19 are diagrams of statuses of a multistable element that is not mounted on a valve body according to this application;
FIG. 20 and FIG. 21 are diagrams of statuses of a multistable element that is mounted on a valve body according to this application;
FIG. 22 and FIG. 23 are diagrams of detecting a first coil winding by a detecting element according to a first detection method of this application;
FIG. 24 and FIG. 25 are diagrams of detecting a second coil winding by a detecting element according to the first detection method of this application;
FIG. 26 and FIG. 27 are diagrams of detecting a second coil winding by a detecting element according to a second detection method of this application;
FIG. 28 and FIG. 29 are diagrams of detecting a first coil winding by a detecting element according to the second detection method of this application;
FIG. 30 is a diagram of a solenoid valve according to a sixth embodiment of this application; and
FIG. 31 is a diagram of a solenoid valve according to a seventh embodiment of this application.

### Reference numerals:

1: solenoid valve; 11: valve body; 111: accommodating cavity; 112: first air intake vent; 113: first air exhaust vent; 114: second air intake vent; 115: second air exhaust vent; 12: valve plug assembly; 121: spacer; 121a: protrusion portion; 122: first sealing element; 123: second sealing element; 124: movable valve plug; 125: movable coil winding; 126: magnetic element; 13: multistable element; 14: first magnet yoke; 141: first mounting groove; 141a: inner wall; 141b: outer wall; 142: first breather hole; 143: tooth groove; 144: recessed portion; 15: second magnet yoke; 151: second mounting groove; 152: second breather hole; 16: first coil winding; 17: second coil winding; 18: elastic element;
2: detecting element;
3: power supply;
Z: height direction; and Y: length direction.

The accompanying drawings herein are incorporated into this specification and constitute a part of this specification, to show embodiments in accordance with this application, and are used, together with this specification, to explain the principle of this application.

### DESCRIPTION OF EMBODIMENTS

To better understand technical solutions of this application, the following describes embodiments of this application in detail with reference to accompanying drawings.

The terms used in embodiments of this application are merely for the purpose of illustrating specific embodiments, and are not intended to limit this application. The terms "a", "said", and "the" of singular forms used in embodiments and the appended claims of this application are also intended to include plural forms, unless otherwise specified in the context clearly.

It should be understood that the term "and/or" in this specification describes only an association relationship between associated objects and represents that three relationships may exist. For example, A and/or B may represent the following three cases: Only A exists, both A and B exist, and only B exists. In addition, the character "/" in this specification generally indicates an "or" relationship between the associated objects.

It should be noted that orientation words such as "up", "down", "left", and "right" described in embodiments of this application are described from angles shown in the accompanying drawings, and should not be understood as a limitation on embodiments of this application. Moreover, in the context, it also should be understood that when it is mentioned that one element is connected "above" or "below" another element, the element can be directly connected "above" or "below" the another element, or may be indirectly connected "above" or "below" the another element by using an intermediate element.

This embodiment provides a device, configured to measure blood pressure. The device may be a blood pressure meter. The device includes at least a power supply 3, a device body, an airbag, an air pump, a pressure sensor, and a solenoid valve 1. The airbag is mounted on the device body, and may be a cuff worn on a measured part such as a wrist or an upper wall. The air pump is mounted on the device body, and the air pump communicates with the airbag, so that the air pump can provide fluid to the airbag and pressurize the airbag, and the fluid is air. The pressure sensor is mounted on the device body, and the pressure sensor is configured to detect pressure of the airbag. The solenoid valve is mounted on the device body, and communicates with the airbag and the air pump, so that when the air pump is faulty and cannot discharge air from the airbag, the airbag can discharge air through the solenoid valve 1, so that the device normally runs. The power supply 3 is mounted on the device body, and is configured to provide electric energy for the air pump, the pressure sensor, and the solenoid valve 1.

To feed back a working status of the solenoid valve 1 in real time, the device further includes a detecting element 2. The detecting element 2 is electrically connected to the solenoid valve 1 to detect whether the solenoid valve 1 normally works, to locate a fault location of the device.

The following describes in detail a specific mechanism of the solenoid valve 1 with reference to the accompanying drawings.

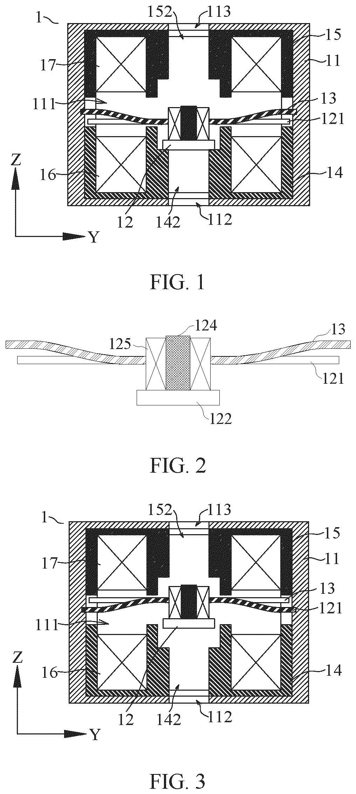

Refer to FIG. 1 to FIG. 14. The solenoid valve 1 includes a valve body 11, a magnet yoke, a coil winding, a valve plug assembly 12, and a multistable element 13. The valve body 11 has an accommodating cavity 111, the magnet yoke is disposed in the accommodating cavity 111, the magnet yoke has a mounting groove, and the coil winding is disposed in the mounting groove. The valve plug assembly 12 is disposed in the accommodating cavity 111. An outer circumferential end of the multistable element 13 is connected to the valve body 11, an inner circumferential end of the multistable element 13 is connected to the valve plug assembly 12, and at least one steady-state position is disposed on the multistable element 13 to position the valve plug assembly 12. When the coil winding is powered on, the magnet yoke can drive the valve plug 124 assembly 12 to move, and the valve plug assembly 12 drives the multistable element 13 to deform to the steady-state position, to position the valve plug assembly 12 to block or open an air path of the solenoid valve 1.

In this embodiment, when the coil winding is powered on, a magnetic field is generated, and the magnetic field polarizes the magnet yoke, so that the magnet yoke generates magnetic force, the magnet yoke can attract or repel the valve plug assembly 12 to move, and the valve plug assembly 12 drives the multistable element 13 to move. The outer circumferential end of the multistable element 13 is fastened to the valve body 11. Therefore, when the multistable element 13 is driven by the valve plug assembly 12 to move, the outer circumferential end of the multistable element 13 is constrained by the valve body 11, thereby implementing buckling deformation, to reach the steady-state position. After the multistable element 13 reaches the steady-state position, the valve plug assembly 12 can be positioned, that is, movement of the valve plug assembly 12 is limited, so that the valve plug assembly 12 remains in a state of blocking the air path of the solenoid valve 1, or the valve plug assembly 12 remains in a state of opening the air path of the solenoid valve 1. In this embodiment, the valve plug assembly 12 is electromagnetically controlled to move to open or block the air path, so that the solenoid valve 1 quickly responds, to discharge air in a timely manner. The multistable element 13 is disposed to limit a position of a valve plug after movement, so that the coil winding can change an air path connectivity status after working (being powered on) for only a short time, thereby reducing energy consumption.

It should be noted that the magnet yoke is generally a soft magnetic material that does not generate a magnetic field (magnetic line) and that only transmits a magnetic line in a magnetic circuit. The magnet yoke is generally made of soft iron, A3 steel, and soft magnetic alloy that have relatively high magnetic permeability.

The multistable element 13 has some elasticity, and can undergo repeated buckling deformation. The multistable element 13 may be made of a plastic or metal material.

The multistable element 13 is divided into a plurality of parts along a circumferential direction of the multistable element 13, and there is an interval between parts of the multistable element 13. The valve plug assembly 12 includes a spacer 121, the spacer 121 is located in the interval, and there is a gap between the adjacent spacer 121 and the multistable element 13. When the coil winding is powered on, the magnet yoke can attract the spacer 121 to move.

In this embodiment, after the coil winding is powered on, a magnetic field is generated, and the magnetic field attracts the spacer 121. The spacer 121 needs to be disposed as close as possible to the coil winding. Therefore, the multistable element 13 is divided into the plurality of parts, and the spacer 121 is disposed in the interval between parts of the multistable element 13, so that the multistable element 13 is not a block between the spacer 121 and the coil winding, and the spacer 121 can be closer to the coil winding, thereby ensuring that the spacer 121 can be attracted to move.

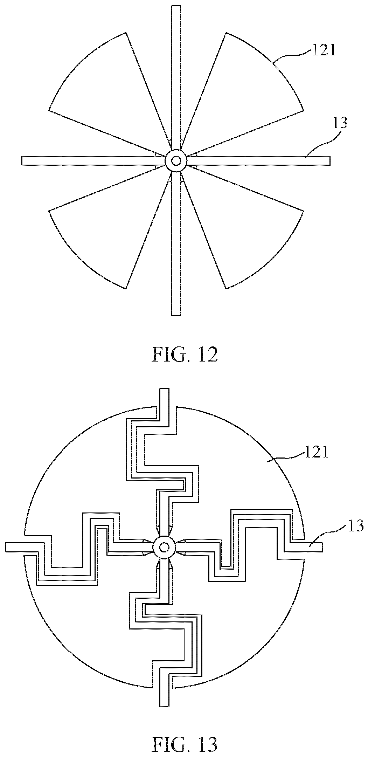

Refer to FIG. 12 and FIG. 13. A shape of the multistable element 13 may be a plurality of linear elastic bands or a plurality of zigzag elastic bands. The zigzag elastic band is similar to a planar spring, and has better elastic performance.

Magnetic attraction force between the magnet yoke and the spacer 121 is closely related to a gap between the magnet yoke and the spacer 121. A smaller gap leads to greater magnetic attraction force. However, a small gap between the magnet yoke and the spacer 121 may hinder airflow flowing, resulting in an air discharge surface. Therefore, a tooth groove 143 is disposed on the magnet yoke. By disposing the tooth groove 143, an area that is of the magnet yoke and that is relative to the spacer 121 may be increased, to improve the magnetic attraction force and increase an airflow rate.

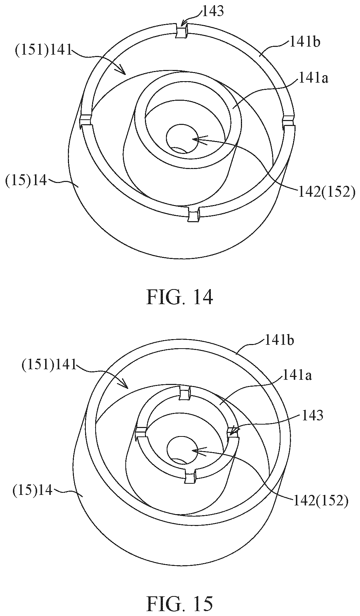

Specifically, refer to FIG. 14. The mounting groove for mounting the coil winding is an annular groove, and the annular groove is provided with an inner wall 141a and an outer wall 141b. The tooth groove 143 may be disposed on an end face of one end that is of the inner wall 141a and that is close to the spacer 121, so that the tooth groove 143 on the end face can circulate airflow and increase an area relative to the spacer 121.

Alternatively, refer to FIG. 15. The mounting groove for mounting the coil winding is an annular groove, and the annular groove is provided with an inner wall 141a and an outer wall 141b. The tooth groove 143 may be disposed on an end face of one end that is of the outer wall 141b and that is close to the spacer 121, so that the tooth groove 143 on the end face can circulate airflow and increase an area relative to the spacer 121.

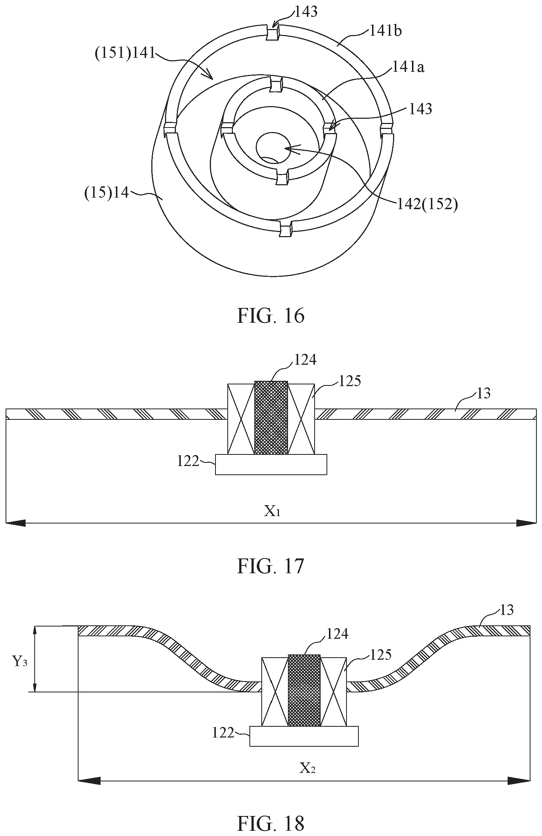

Alternatively, refer to FIG. 16. The mounting groove for mounting the coil winding is an annular groove, and the annular groove is provided with an inner wall 141a and an outer wall 141b. The tooth groove 143 may be disposed on end faces of ends that are of both the inner wall 141a and the outer wall 141b and that are close to the spacer 121, so that the tooth groove 143 on the end face can circulate airflow and increase an area relative to the spacer 121.

Refer to FIG. 17 to FIG. 21. The bistable element needs to meet the following requirements:
Refer to FIG. 17. FIG. 17 shows that a transverse distance of the multistable element 13 before being processed is X1.

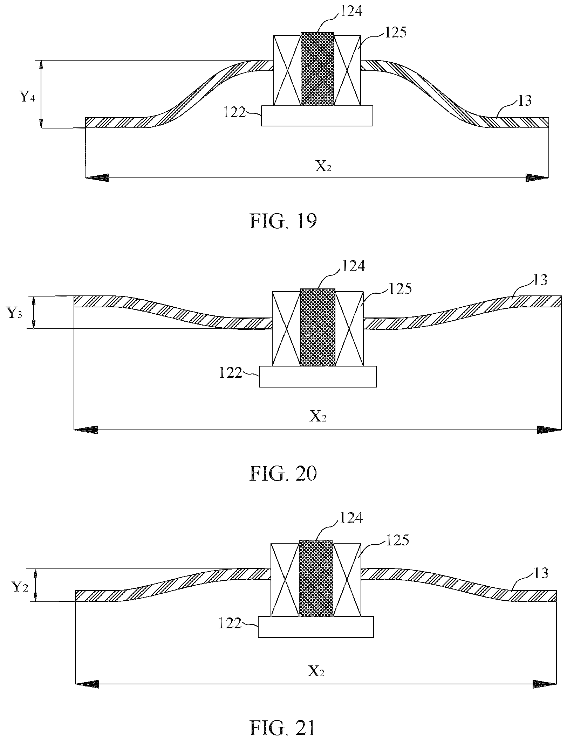

Refer to FIG. 18 and FIG. 19. Before the multistable element 13 is mounted on the valve body 11, in a buckling deformation process of the multistable element 13, the transverse distance of the bistable element is shortened from X1 to X2, an upward buckling deformation distance of the multistable element 13 is Y1, and a downward buckling deformation distance of the multistable element 13 is Y2.

Refer to FIG. 20 and FIG. 21. After the multistable element 13 is mounted on the valve body 11, in a buckling deformation process of the multistable element 13, the transverse distance of the bistable element is shortened from X1 to X2, an upward buckling deformation distance of the multistable element 13 is Y3, and a downward buckling deformation distance of the multistable element 13 is Y4.

Y3 < Y1 and Y4 < Y2 need to be met, that is, the valve body 11 limits a buckling deformation amount after the multistable element 13 is mounted on the valve body 11 to be less than a buckling deformation amount before the multistable element 13 is mounted on the valve body 11, so that the multistable element 13 of the valve body 11 can still have a deformation trend after being mounted, limit movement of the valve plug assembly 12, and position the valve plug assembly 12 in the state of blocking the air path or in the state of opening the air path.

Refer to FIG. 1 to FIG. 3. FIG. 1 is a diagram of the solenoid valve 1 in a closed state according to a first specific embodiment. FIG. 2 is a diagram of a structure of the valve plug assembly 12 according to the first embodiment. FIG. 3 is a diagram of the solenoid valve 1 in an open state according to the first specific embodiment. In this embodiment, the magnet yoke includes a first magnet yoke 14 and a second magnet yoke 15. Along a height direction Z of the solenoid valve 1, the first magnet yoke 14 is disposed on one side of the valve plug assembly 12, and the second magnet yoke 15 is disposed on the other side of the valve plug assembly 12. The first magnet yoke 14 has a first mounting groove 141, and the second magnet yoke 15 has a second mounting groove 151. The coil winding includes a first coil winding 16 and a second coil winding 17. The first coil winding 16 is disposed in the first mounting groove 141, and the second coil winding 17 is disposed in the second mounting groove 151. When the first coil winding 16 is powered on, the first magnet yoke 14 can attract the spacer 121, and the spacer 121 drives the multistable element 13 to deform to a first steady-state position. When the second coil winding 17 is powered on, the second magnet yoke 15 can attract the spacer 121, and the spacer 121 drives the multistable element 13 to deform to a second steady-state position.

The valve body 11 further has a first air intake vent 112 and a first air exhaust vent 113. The first magnet yoke 14 further has a first breather hole 142. The first mounting groove 141 is disposed around an outer ring of the first breather hole 142, an opening of the first breather hole 142 is lower than an opening of the first mounting groove 141, and the first breather hole 142 communicates with the first air intake vent 112. The second magnet yoke 15 further has a second breather hole 152. The second mounting groove 151 is disposed around an outer ring of the second breather hole 152, an opening of the second breather hole 152 is lower than an opening of the second mounting groove 151, and the second breather hole 152 communicates with the first air exhaust vent 113. The valve plug assembly 12 includes a first sealing element 122, and the first sealing element 122 is disposed close to the first breather hole 142.

When the first coil winding 16 is powered on, the first magnet yoke 14 can attract the spacer 121, and the spacer 121 drives the multistable element 13 to deform to the first steady-state position, so that the first sealing element 122 seals the first breather hole 142 to block the air path. When the second coil winding 17 is powered on, the second magnet yoke 15 can attract the spacer 121, and the spacer 121 drives the multistable element 13 to deform to the second steady-state position, so that the first sealing element 122 is away from the first breather hole, the spacer 121 abuts against the second magnet yoke 15, and the first air intake vent 112 communicates with the first air exhaust vent 113 to open the air path.

In a specific solution, the first breather hole 142, the second breather hole 152, and the accommodating cavity 111 communicate with each other, and the first air intake vent 112 and the first air exhaust vent 113 are disposed opposite to each other along the height direction Z of the solenoid valve 1.

Refer to FIG. 1. When the first coil winding 16 is powered on, the second coil winding 17 is in a power-off state, the first coil winding 16 generates a magnetic field, and the magnetic field polarizes the first magnet yoke 14 to generate magnetic force, so that the first magnet yoke 14 can attract the spacer 121 to move in a direction close to the first magnet yoke 14, the spacer 121 drives the first sealing element 122 to move, and the first sealing element 122 moves to a position at which the first breather hole 142 is blocked. In addition, the spacer 121 drives the multistable element 13 to deform to the first steady-state position, so that the multistable element 13 presses the first sealing element 122 to always maintain a state of blocking the first breather hole 142, thereby closing the air path of the solenoid valve 1.

Refer to FIG. 3. When the second coil winding 17 is powered on, the first coil winding 16 is in a power-off state, the second coil winding 17 generates a magnetic field, and the magnetic field polarizes the second magnet yoke 15 to generate magnetic force, so that the second magnet yoke 15 can attract the spacer 121 to move in a direction close to the second magnet yoke 15, and the spacer 121 drives the first sealing element 122 to move away from the first breather hole 142. When the spacer 121 moves to abut against the second magnet yoke 15, the spacer 121 drives the multistable element 13 to deform to the second steady-state position, so that the multistable element 13 presses the spacer 121 to maintain a state of abutting against the second magnet yoke 15, and the first sealing element 122 maintains a state of being away from the first breather hole 142. The first breather hole 142, the accommodating cavity 111, and the second breather hole 152 communicate with each other, so that the first air intake vent 112 communicates with the first air exhaust vent 113, thereby opening the air path of the circuit valve.

Along the height direction Z of the solenoid valve 1, a projection surface of the first sealing element 122 can cover a projection surface of the first breather hole 142, so that the first sealing element 122 can block the first breather hole 142.

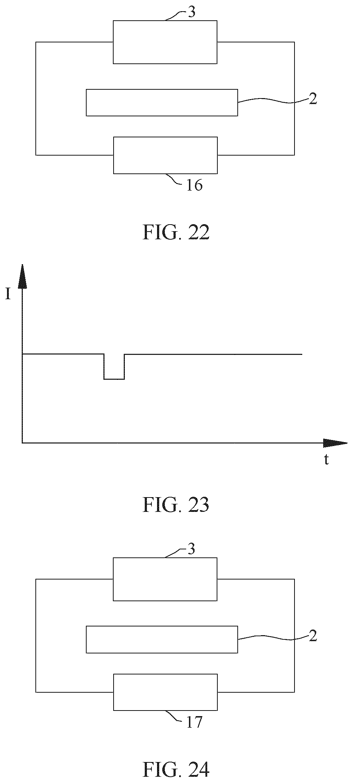

Refer to FIG. 22 to FIG. 25. In this embodiment, the valve plug assembly 12 further includes a movable valve plug 124 and a movable coil winding 125. The movable coil winding 125 is disposed around an outer ring of the movable valve plug 124. When the first magnet yoke 14 attracts the movable valve plug 124 to move, the movable valve plug 124 drives the movable coil winding 125 to move in a direction close to the first coil winding 16, so that a magnetic field generated by the movable coil winding 125 interacts with a magnetic field generated by the first coil winding 16, to detect a signal change of the first coil winding 16, and determine whether the solenoid valve 1 normally works. When the second magnet yoke 15 attracts the movable valve plug 124 to move, the movable valve plug 124 drives the movable coil winding 125 to move in a direction close to the second coil winding 17, so that a magnetic field generated by the movable coil winding 125 interacts with a magnetic field generated by the second coil winding 17, to detect a signal change of the second coil winding 17, and determine whether the solenoid valve 1 normally works.

In a specific solution, the movable valve plug 124 and the movable coil winding 125 are disposed in a middle region of the spacer 121 and the multistable element 13, the first sealing element 122 is disposed at one end of the movable valve plug 124, and a material of the movable valve plug 124 may be iron.

The movable coil winding 125 is a closed circuit. When the movable coil winding 125 is driven to move, a magnetic induction line is cut to generate an induced current, and the induced current generates an induced magnetic field.

Refer to FIG. 22. When the first coil winding 16 is powered on, the first coil winding 16 generates a magnetic field, so that the first magnet yoke 14 generates magnetic force and can attract the movable valve plug 124 to move towards the first magnet yoke 14. The movable valve plug 124 drives the movable coil winding 125 to move in a direction close to the first coil winding 16. A magnetic field generated by the movable coil winding 125 in a moving process interacts with a magnetic field generated by the first coil winding 16, and affects an electrical signal of the first coil winding 16. Therefore, a detecting element 2 is used to detect a change of the electrical signal (for example, a current, a voltage, or power) of the first coil winding 16, to determine whether the first sealing element 122 moves in a direction close to the first breather hole 142, to determine whether the solenoid valve 1 normally works. Specifically, as shown in FIG. 23, t represents a time, I represents an electrical signal, and a curve represents a change of the electrical signal of the first coil winding 16 detected by the detecting element 2 when the movable coil winding 125 moves.

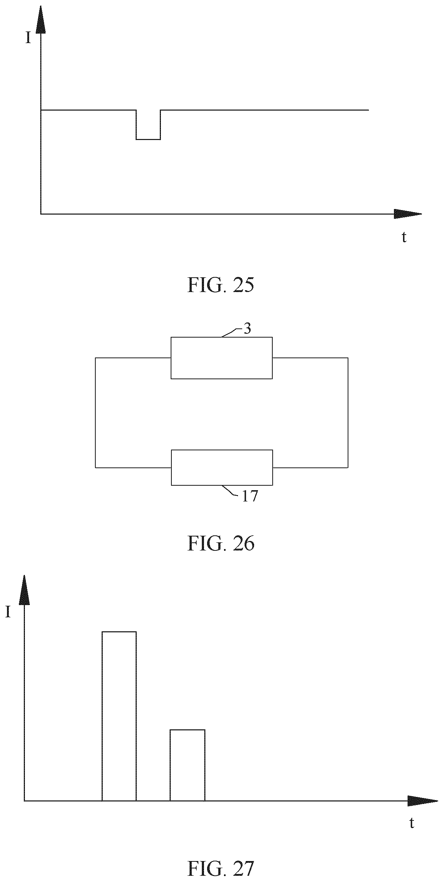

Refer to FIG. 24. When the second coil winding 17 is powered on, the second coil winding 17 generates a magnetic field, so that the second magnet yoke 15 generates magnetic force and can attract the movable valve plug 124 to move towards the second magnet yoke 15. The movable valve plug 124 drives the movable coil winding 125 to move in a direction close to the second coil winding 17. A magnetic field generated by the movable coil winding 125 in a moving process interacts with a magnetic field generated by the second coil winding 17, and affects an electrical signal of the second coil winding 17. Therefore, the detecting element 2 is used to detect a change of the electrical signal (for example, a current, a voltage, or power) of the second coil winding 17, to determine whether the first sealing element 122 moves in a direction away from the first breather hole 142, to determine whether the solenoid valve 1 normally works. Specifically, as shown in FIG. 25, t represents a time, I represents an electrical signal, and a curve represents a change of the electrical signal of the second coil winding 17 detected by the detecting element 2 when the movable coil winding 125 moves.

Refer to FIG. 26 to FIG. 29. Another detection method may alternatively be used in this embodiment. When the first coil winding 16 is powered on and the first magnet yoke 14 attracts the movable valve plug 124 to move, the second coil winding 17 can generate a magnetic field, to detect a signal change of the second coil winding 17, and determine whether the first coil winding 16 normally works. When the second coil winding 17 is powered on and the second magnet yoke 15 attracts the movable valve plug 124 to move, the first coil winding 16 can generate a magnetic field, to detect a signal change of the first coil winding 16, and determine whether the second coil winding 17 normally works.

Refer to FIG. 26. When the first coil winding 16 is powered on, a magnetic field is generated. As a result, the second coil winding 17 also generates a magnetic field, the magnetic field generates an electrical signal, and the detecting element 2 is used to detect a change of the electrical signal of the second coil winding 17. In addition, a magnetic field generated by the movable coil winding 125 when moving also causes a change of the electrical signal of the second coil. The detecting element 2 is used to detect the change of the electrical signal of the second coil winding 17, to determine whether the first coil winding 16 normally works, and determine whether the first sealing element 122 moves in a direction close to the first breather hole 142, to determine whether the solenoid valve 1 normally works. Specifically, as shown in FIG. 27, t represents a time, I represents an electrical signal, and a curve represents a change of the electrical signal of the second coil winding 17 detected by the detecting element 2 when the first coil winding 16 is powered on and the movable coil winding 125 moves.

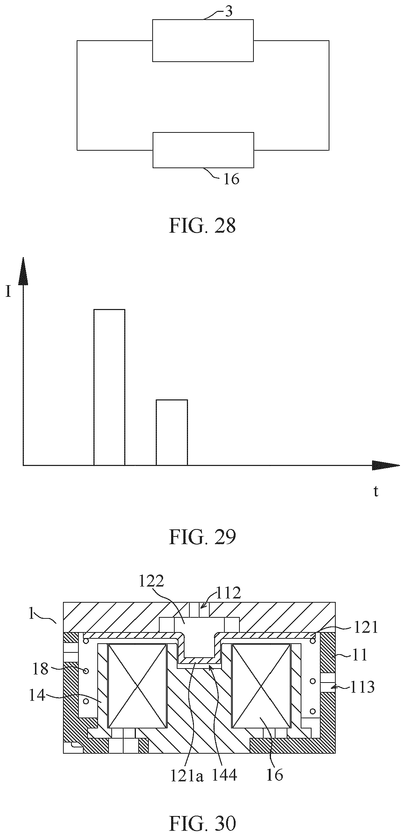

Refer to FIG. 28. When the second coil winding 17 is powered on, a magnetic field is generated. As a result, the first coil winding 16 also generates a magnetic field, the magnetic field generates an electrical signal, and the detecting element 2 is used to detect a change of the electrical signal of the first coil winding 16. In addition, a magnetic field generated by the movable coil winding 125 when moving also causes a change of the electrical signal of the first coil. The detecting element 2 is used to detect the change of the electrical signal of the first coil winding 16, to determine whether the second coil winding 17 normally works, and determine whether the first sealing element 122 moves in a direction away from the first breather hole 142, to determine whether the solenoid valve 1 normally works. Specifically, as shown in FIG. 29, t represents a time, I represents an electrical signal, and a curve represents a change of the electrical signal of the first coil winding 16 detected by the detecting element 2 when the second coil winding 17 is powered on and the movable coil winding 125 moves.

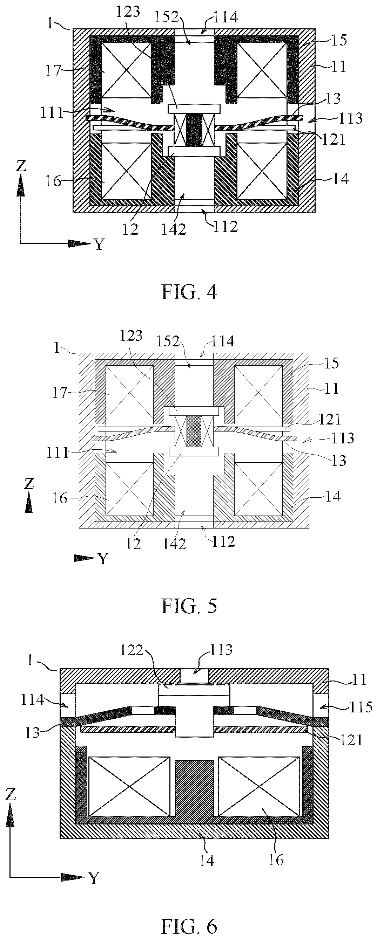

Refer to FIG. 4 and FIG. 5. FIG. 4 and FIG. 5 are diagrams of a structure of the solenoid valve 1 according to a second specific embodiment. In this embodiment, different from the first embodiment, the solenoid valve 1 is a three-way valve, and the valve body 11 further has a first air intake vent 112, a second air intake vent 114, and a first air exhaust vent 113. The first magnet yoke 14 further has a first breather hole 142, the first breather hole 142 communicates with the first air intake vent 112, the second magnet yoke 15 further has a second breather hole 152, and the second breather hole 152 communicates with the second air intake vent 114. The valve plug assembly 12 further includes a first sealing element 122 and a second sealing element 123, the first sealing element 122 is disposed close to the first breather hole 142, and the second sealing element 123 is disposed close to the second breather hole 152. When the first coil winding 16 is powered on, the first magnet yoke 14 can attract the spacer 121, and the spacer 121 drives the multistable element 13 to deform to the first steady-state position, so that the first sealing element 122 seals the first breather hole 142, and the second air intake vent 114 communicates with the first air exhaust vent 113. When the second coil winding 17 is powered on, the second magnet yoke 15 can attract the spacer 121, and the spacer 121 drives the multistable element 13 to deform to the second steady-state position, so that the second sealing element 123 seals the second breather hole 152, and the first air intake vent 112 communicates with the first air exhaust vent 113.

In a specific solution, along the height direction Z of the solenoid valve 1, the first air intake vent 112 and the second air intake vent 114 are disposed opposite to each other, and the first air exhaust vent 113 is disposed on one side of the first air intake vent 112 and the second air intake vent 114.

Refer to FIG. 4. When the first coil winding 16 is powered on, the second coil winding 17 is in a power-off state, the first coil winding 16 generates a magnetic field, and the magnetic field polarizes the first magnet yoke 14 to generate magnetic force, so that the first magnet yoke 14 can attract the spacer 121 to move in a direction close to the first magnet yoke 14, the spacer 121 drives the first sealing element 122 to move, and the first sealing element 122 moves to a position at which the first breather hole 142 is blocked, to block the first air intake vent 112. In addition, the spacer 121 drives the multistable element 13 to deform to the first steady-state position, so that the multistable element 13 presses the first sealing element 122 to always maintain a state of blocking the first air intake vent, thereby implementing communication between the second air intake vent 114 and the first air exhaust vent 113.

Refer to FIG. 5. When the second coil winding 17 is powered on, the first coil winding 16 is in a power-off state, the second coil winding 17 generates a magnetic field, and the magnetic field polarizes the second magnet yoke 15 to generate magnetic force, so that the second magnet yoke 15 can attract the spacer 121 to move in a direction close to the second magnet yoke 15, the spacer 121 drives the second sealing element 123 to move, and the second sealing element 123 moves to a position at which the second breather hole 152 is blocked, to block the second air intake vent 114. In addition, the spacer 121 drives the multistable element 13 to deform to the second steady-state position, so that the multistable element 13 presses the second sealing element 123 to always maintain a state of blocking the second air intake vent, thereby implementing communication between the first air intake vent 112 and the first air exhaust vent 113.

A method for detecting the solenoid valve 1 by using the detecting element 2 in this embodiment is the same as that in the first embodiment, and details are not described herein.

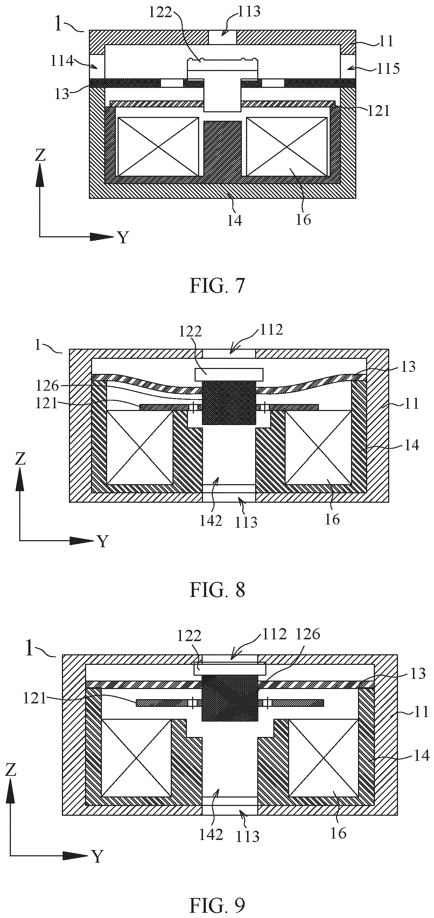

Refer to FIG. 6 and FIG. 7. FIG. 6 and FIG. 7 are diagrams of a structure of the solenoid valve 1 according to a third specific embodiment. In this embodiment, the solenoid valve 1 is a normally closed solenoid valve 1. The valve body 11 includes a first air intake vent 112, a first air exhaust vent 113, and a second air exhaust vent 115. The valve plug assembly 12 includes a first sealing element 122, and the first sealing element 122 is connected to the multistable element 13. When the coil winding is powered on, the magnet yoke can attract the spacer 121, and the spacer 121 drives the multistable element 13 to move and deform, so that the spacer 121 abuts against the magnet yoke, and the first air intake vent 112, the first air exhaust vent 113, and the second air exhaust vent 115 communicate with each other. When the coil winding is powered off, the multistable element 13 deforms and rebounds to a second steady-state position, and drives the first sealing element 122 to seal the first air intake vent 112. When the coil winding is powered on, a buckling deformation distance of the multistable element 13 is less than Y4.

In a specific solution, the first air intake vent 112 penetrates a side wall of the valve body 11 along the height direction Z of the solenoid valve 1, the first air exhaust vent 113 and the second air exhaust vent 115 are respectively disposed at two ends of the first air intake vent 112, and the first air exhaust vent 113 and the second air exhaust vent 115 are disposed opposite to each other.

In this embodiment, compared with the first embodiment, one coil winding and one magnet yoke are disposed, and no movable coil winding 125 is disposed.

Refer to FIG. 6. When the coil winding is not powered on, the multistable element 13 is at the second steady-state position, and the multistable element 13 presses the first sealing element 122 to seal the first air intake vent 112, thereby closing the air path of the solenoid valve 1.

Refer to FIG. 7. When the coil winding is powered on, the coil winding generates a magnetic field, and the magnet yoke is magnetized to generate magnetic force, so that the magnet yoke attracts the spacer 121 to move in a direction close to the magnet yoke. The spacer 121 drives the first sealing element 122 to move away from the first air intake vent 112, and the spacer 121 moves to abut against the magnet yoke, so that the first air intake vent 112 communicates with the first air exhaust vent 113 and the second air exhaust vent 115, thereby opening the solenoid valve 1. In addition, the spacer 121 drives the multistable element 13 to deform, and in this case, a deformation distance of the multistable element 13 is less than a distance of deformation of the multistable element 13 to the second steady-state position.

Refer to FIG. 6. When the coil winding is powered off, the magnetic field disappears. Because the deformation distance of the multistable element 13 when the coil winding is powered on is less than the distance of deformation of the multistable element 13 to the second steady-state position, in this case, the multistable element 13 deforms and rebounds towards the second steady-state position until the first sealing element 122 reaches the top of the valve body 11 to block the first air intake vent 112, so that the solenoid valve 1 remains in a closed state.

Refer to FIG. 8 and FIG. 9. FIG. 8 and FIG. 9 are diagrams of a structure of the solenoid valve 1 according to a fourth specific embodiment. In this embodiment, the solenoid valve 1 is a normally open solenoid valve 1. The valve body 11 includes a first air intake vent 112 and a first air exhaust vent 113. A first breather hole 142 is disposed on the magnet yoke, and the first breather hole 142 communicates with the first air exhaust vent 113. The valve plug assembly 12 further includes a first sealing element 122 and a magnetic element 126, the first sealing element 122 is disposed at one end that is of the magnetic element 126 and that is close to the first air intake vent 112, and the magnetic element 126 can magnetize the spacer 121. When the coil winding is powered off, the magnet yoke attracts the spacer 121, and the spacer 121 drives the multistable element 13 to move and deform to a first steady-state position, so that the spacer 121 abuts against the magnet yoke, and the first air intake vent 112 communicates with the first air exhaust vent 113 through the first breather hole 142, to open the air path. When the coil winding is powered on, under an action of a magnetic field, the magnet yoke repels the spacer 121, and the spacer 121 drives the first sealing element 122 to seal the first air intake vent 112, to block the air path.

In a specific solution, the first air intake vent 112 and the first air exhaust vent 113 are disposed opposite to each other along the height direction Z of the solenoid valve 1.

In this embodiment, compared with the first embodiment, one coil winding and one magnet yoke are disposed, no movable coil winding 125 is disposed, the movable valve plug 124 is replaced with the magnetic element 126, and the magnetic element 126 is a permanent magnet.

Refer to FIG. 8. When the coil winding is powered off, the magnetic element 126 magnetizes the spacer 121, and the magnet yoke attracts the spacer 121 to move in a direction close to the magnet yoke, so that the spacer 121 is finally attached to the magnet yoke. The spacer 121 drives the first sealing element 122 to move away from the first air intake vent 112, and the first air intake vent 112 communicates with the first air exhaust vent 113, so that the air path of the solenoid valve 1 remains in an open state. In addition, the spacer 121 drives the multistable element 13 to deform to the first steady-state position, and the multistable element 13 is in a stretched state.

Refer to FIG. 9. When the coil winding is powered on, the coil winding generates a magnetic field. The magnetic field generated by the coil winding is opposite to a magnetic field of the magnetic element 126, and the spacer 121 and the magnetic element 126 are repelled to move in a direction away from the magnet yoke. Rebound force of the multistable element 13 and repelling force generated by the coil winding press the first sealing element 122 onto the valve body 11 through the spacer 121, and the first air intake vent 112 is blocked, thereby closing the air path of the solenoid valve 1.

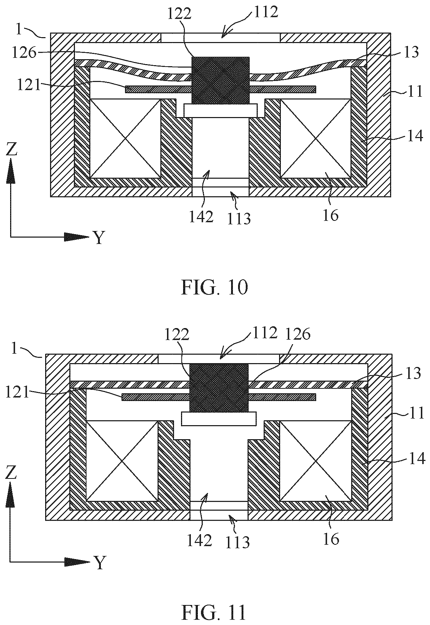

Refer to FIG. 10 and FIG. 11. FIG. 10 and FIG. 11 are diagrams of a structure of the solenoid valve 1 according to a fifth specific embodiment. In this embodiment, the solenoid valve 1 is a normally closed solenoid valve 1. The valve body 11 includes a first air intake vent 112 and a first air exhaust vent 113. A first breather hole 142 is disposed on the magnet yoke, and the first breather hole 142 communicates with the first air exhaust vent 113. The valve plug assembly 12 further includes a first sealing element 122 and a magnetic element 126, the first sealing element 122 is disposed at one end that is of the magnetic element 126 and that is close to the first air exhaust vent 113, and the magnetic element 126 can magnetize the spacer 121. When the coil winding is powered off, the magnet yoke attracts the spacer 121, the spacer 121 drives the first sealing element 122 to seal the first breather hole 142, and the multistable element 13 moves and deforms to a second steady-state position, to block the air path. When the coil winding is powered on, under an action of a magnetic field, the magnet yoke repels the spacer 121, and the spacer 121 drives the multistable element 13 to deform, so that the first sealing element 122 is away from the first breather hole 142, and the first air intake vent 112 communicates with the first air exhaust vent 113 through the first breather hole 142, to open the air path.

In a specific solution, the first air intake vent 112 and the first air exhaust vent 113 are disposed opposite to each other along the height direction Z of the solenoid valve 1.

In this embodiment, compared with the first embodiment, one coil winding and one magnet yoke are disposed, no movable coil winding 125 is disposed, the movable valve plug 124 is replaced with the magnetic element 126, and the magnetic element 126 is a permanent magnet.

Refer to FIG. 10. When the coil winding is powered off, the magnetic element 126 magnetizes the spacer 121, and the magnet yoke attracts the spacer 121 to move in a direction close to the magnet yoke, so that the spacer 121 is finally attached to the magnet yoke. The spacer 121 drives the first sealing element 122 to block the first breather hole 142, to block the first air exhaust vent 113, so that the air path of the solenoid valve 1 remains in a closed state. In addition, the spacer 121 drives the multistable element 13 to deform to the second steady-state position, and the multistable element 13 is in a stretched state.

Refer to FIG. 11. When the coil winding is powered on, the coil winding generates a magnetic field. The magnetic field generated by the coil winding is opposite to a magnetic field of the magnetic element 126, and the spacer 121 and the magnetic element 126 are repelled to move in a direction away from the magnet yoke. Rebound force of the multistable element 13 and repelling force generated by the coil winding drive the first sealing element 122 through the spacer 121 to move in a direction away from the first breather hole 142, so that the first air intake vent 112 communicates with the first air exhaust vent 113 through the first breather hole 142, thereby opening the air path of the solenoid valve 1.

Along a height direction Z of the solenoid valve 1, a cross-sectional area of the first air intake vent 112 is greater than a cross-sectional area of the magnetic element 126, so that when the magnetic element 126 moves to be close to the first air intake vent 112, the first air intake vent 112 is not blocked, thereby ensuring communication between the first air intake vent 112 and the first air exhaust vent 113.

Refer to FIG. 30. FIG. 30 is a diagram of a structure of the solenoid valve 1 according to a sixth specific embodiment. In this embodiment, the solenoid valve 1 is a normally closed solenoid valve 1. This embodiment differs from the first embodiment in that the movable first coil winding 16 is removed, the multistable element 13 is replaced with an elastic element 18, and a set of first coil winding 16 and first magnet yoke 14 is disposed. The valve plug assembly 12 includes a spacer 121 and a first sealing element 122, and the spacer 121 is connected to the first sealing element 122. The elastic element 18 is disposed between the spacer 121 and an inner bottom wall of the valve body 11. The valve body 11 has a first air intake vent 112 and a first air exhaust vent 113, and the first sealing element 122 is disposed close to the first air intake vent 112. When the first coil winding 16 is powered off, under elastic force of the elastic element 18, the spacer 121 presses the first sealing element 122 to seal the first air intake vent 112, to block the air path. When the first coil winding 16 is powered on, the first magnet yoke 14 attracts the spacer 121, the spacer 121 overcomes the elastic force of the elastic element 18 to drive the first sealing element 122 to move in a direction away from the first air intake vent 112, and the first air intake vent 112 communicates with the first air exhaust vent 113, to open the air path.

In a specific solution, the first air intake vent 112 penetrates a side wall of the valve body 11 along a height direction Z of the solenoid valve 1, and the first air exhaust vent 113 is disposed on one side of the first air intake vent along a length direction Y of the solenoid valve 1. The elastic element 18 may be a spring. One end of the spring is connected to the spacer 121, the other end is connected to an inner bottom wall of the valve body 11, and the spring is mounted in the accommodating cavity 111 in a pre-compressed state.

When the first coil winding 16 is powered off, the elastic element 18 can press the spacer 121 through elastic force of the elastic element 18, and the spacer 121 abuts against an inner side wall of the valve body 11, so that the spacer 121 presses the first sealing element 122 to block the first air intake vent 112, and the air path of the solenoid valve 1 remains in a closed state.

When the first coil winding 16 is powered on, a magnetic field is generated, and the first magnet yoke 14 is magnetized to generate magnetic force. The first magnet yoke 14 attracts the spacer 121 to overcome the elastic force of the elastic element 18 and move in a direction close to the first magnet yoke 14. The spacer 121 drives the first sealing element 122 to move away from the first air intake vent 112, so that the first air intake vent 112 communicates with the first air exhaust vent 113, thereby opening the air path of the solenoid valve 1.

A recessed portion 144 is disposed on the first magnet yoke 14, a protrusion portion 121a corresponding to the recessed portion 144 is disposed on the spacer 121, and at least a part of the protrusion portion 121a is disposed in the recessed portion 144. Along a height direction Z of the solenoid valve 1, the protrusion portion 121a can move along the recessed portion 144. In a moving process of the spacer 121, the protrusion portion 121a can move in the recessed portion 144. By disposing the protrusion portion 121a and the recessed portion 144, a contact area between the spacer 121 and the first magnet yoke 14 is increased, thereby enhancing magnetic attraction force.

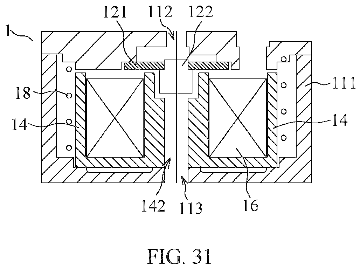

Refer to FIG. 31. FIG. 31 is a diagram of a structure of the solenoid valve 1 according to a seventh specific embodiment. In this embodiment, the solenoid valve 1 is a normally open solenoid valve 1. This embodiment differs from the first embodiment in that the movable first coil winding 16 is removed, the multistable element 13 is replaced with an elastic element 18, and a set of first coil winding 16 and first magnet yoke 14 is disposed. The valve plug assembly 12 includes a spacer 121 and a first sealing element 122, and the spacer 121 is connected to the first sealing element 122. The multistable element 13 is disposed between the spacer 121 and an inner bottom wall of the valve body 11. The valve body 11 has a first air intake vent 112 and a first air exhaust vent 113, and the first sealing element 122 is disposed close to the first air exhaust vent 113. A first breather hole 142 is disposed on the first magnet yoke 14, and the first breather hole 142 communicates with the first air exhaust vent 113. When the first coil winding 16 is powered off, the spacer 121 is pressed under elastic force of the multistable element 13, so that the first sealing element 122 is away from the first breather hole 142, and the first air intake vent 112 communicates with the first air exhaust vent 113 through the first breather hole 142, to open the air path. When the first coil winding 16 is powered on, the first magnet yoke 14 attracts the spacer 121, and the spacer 121 overcomes the elastic force of the multistable element 13 to drive the first sealing element 122 to seal the first breather hole 142, to block the air path.

In a specific solution, the first air intake vent 112 and the first air exhaust vent 113 are disposed opposite to each other along the height direction Z of the solenoid valve 1. The elastic element 18 may be a spring. One end of the spring is connected to the spacer 121, the other end is connected to an inner bottom wall of the valve body 11, and the spring is mounted in the accommodating cavity 111 in a pre-compressed state.

When the first coil winding 16 is powered off, the elastic element 18 can press the spacer 121 through elastic force of the elastic element 18, so that the spacer 121 abuts against the valve body 11. In this case, the first sealing element 122 is away from the first breather hole 142, and the air path of the solenoid valve 1 remains in an open state.

When the first coil winding 16 is powered on, a magnetic field is generated, and the first magnet yoke 14 is magnetized to generate magnetic force. The first magnet yoke 14 attracts the spacer 121 to overcome the elastic force of the elastic element 18 and move in a direction close to the first magnet yoke 14. The spacer 121 drives the first sealing element 122 to block the first breather hole 142, thereby closing the air path of the solenoid valve 1.

In the foregoing embodiments, the first sealing element 122 and the second sealing element 123 may be rubber plugs.

The foregoing describes merely preferred embodiments of this application, which are not intended to limit this application. For a person skilled in the art, various modifications and changes may be made in this application. Any modification, equivalent replacement, or improvement made without departing from the principle of this application shall fall within the protection scope of this application.

## Claims

1. **A solenoid** valve, wherein the solenoid valve comprises:
a valve body, wherein the valve body has an accommodating cavity;
a magnet yoke, wherein the magnet yoke is disposed in the accommodating cavity, and the magnet yoke has a mounting groove;
a coil winding, wherein the coil winding is disposed in the mounting groove;
a valve plug assembly, wherein the valve plug assembly is disposed in the accommodating cavity and can move relative to the valve body, and the valve plug assembly comprises a movable coil winding; and
a multistable element, wherein an outer circumferential end of the multistable element is connected to the valve body, an inner circumferential end of the multistable element is connected to the valve plug assembly, and at least one steady-state position is disposed on the multistable element to position the valve plug assembly, wherein
when the coil winding is powered on, the magnet yoke can drive the valve plug assembly to move, and the valve plug assembly drives the multistable element to deform to the steady-state position, so that the valve plug assembly blocks or opens an air path of the solenoid valve; and
when the valve plug assembly moves, the movable coil winding can generate a magnetic field, and the magnetic field generated by the movable coil can interact with a magnetic field generated by the coil winding, to detect a signal change of the coil winding.

2. The solenoid valve according to claim 1, wherein before the multistable element is mounted on the valve body, an upward buckling deformation distance of the multistable element is Y1, and a downward buckling deformation distance of the multistable element is Y2; and
after the multistable element is mounted on the valve body, an upward buckling deformation distance of the multistable element is Y3, and a downward buckling deformation distance of the multistable element is Y4, wherein
Y3 < Y1 and Y4 < Y2 are met, so that the multistable element positions the valve plug assembly.

3. The solenoid valve according to claim 2, wherein the multistable element is divided into a plurality of parts along a circumferential direction of the multistable element, and there is an interval between parts of the multistable element;
the valve plug assembly comprises a spacer, the spacer is located in the interval, and there is a gap between the adjacent spacer and the multistable element; and
when the coil winding is powered on, the magnet yoke can attract the spacer to move.

4. The solenoid valve according to claim 3, wherein the magnet yoke comprises a first magnet yoke and a second magnet yoke; along a height direction of the solenoid valve, the first magnet yoke is disposed on one side of the valve plug assembly, and the second magnet yoke is disposed on the other side of the valve plug assembly;
the first magnet yoke has a first mounting groove, and the second magnet yoke has a second mounting groove;
the coil winding comprises a first coil winding and a second coil winding, the first coil winding is disposed in the first mounting groove, and the second coil winding is disposed in the second mounting groove;
when the first coil winding is powered on, the first magnet yoke can attract the spacer, and the spacer drives the multistable element to deform to a first steady-state position; and
when the second coil winding is powered on, the second magnet yoke can attract the spacer, and the spacer drives the multistable element to deform to a second steady-state position.

5. The solenoid valve according to claim 4, wherein the valve body further has a first air intake vent and a first air exhaust vent;
the first magnet yoke further has a first breather hole, the first breather hole communicates with the first air intake vent, the second magnet yoke further has a second breather hole, and the second breather hole communicates with the first air exhaust vent;
the valve plug assembly comprises a first sealing element, and the first sealing element is disposed close to the first breather hole;
when the first coil winding is powered on, the first magnet yoke can attract the spacer, and the spacer drives the multistable element to deform to the first steady-state position, so that the first sealing element seals the first breather hole to block the air path; and
when the second coil winding is powered on, the second magnet yoke can attract the spacer, and the spacer drives the multistable element to deform to the second steady-state position, so that the first sealing element is away from the first breather hole, the spacer abuts against the second magnet yoke, and the first air intake vent communicates with the first air exhaust vent to open the air path.

6. The solenoid valve according to claim 4, wherein the valve body further has a first air intake vent, a second air intake vent, and a first air exhaust vent;
the first magnet yoke further has a first breather hole, the first breather hole communicates with the first air intake vent, the second magnet yoke further has a second breather hole, and the second breather hole communicates with the second air intake vent;
the valve plug assembly further comprises a first sealing element and a second sealing element, the first sealing element is disposed close to the first breather hole, and the second sealing element is disposed close to the second breather hole;
when the first coil winding is powered on, the first magnet yoke can attract the spacer, and the spacer drives the multistable element to deform to the first steady-state position, so that the first sealing element seals the first breather hole, and the second air intake vent communicates with the first air exhaust vent; and
when the second coil winding is powered on, the second magnet yoke can attract the spacer, and the spacer drives the multistable element to deform to the second steady-state position, so that the second sealing element seals the second breather hole, and the first air intake vent communicates with the first air exhaust vent.

7. The solenoid valve according to claim 5 or 6, wherein the valve plug assembly further comprises a movable valve plug, and the movable coil winding is disposed around an outer ring of the movable valve plug;
when the first magnet yoke attracts the movable valve plug to move, the movable valve plug drives the movable coil winding to move in a direction close to the first coil winding, so that a magnetic field generated by the movable coil winding interacts with a magnetic field generated by the first coil winding, to detect a signal change of the first coil winding, and determine whether the solenoid valve normally works; and
when the second magnet yoke attracts the movable valve plug to move, the movable valve plug drives the movable coil winding to move in a direction close to the second coil winding, so that a magnetic field generated by the movable coil winding interacts with a magnetic field generated by the second coil winding, to detect a signal change of the second coil winding, and determine whether the solenoid valve normally works.

8. The solenoid valve according to claim 5 or 6, wherein the valve plug assembly further comprises a movable valve plug, and the movable coil winding is disposed around an outer ring of the movable valve plug;
when the first coil winding is powered on and the first magnet yoke attracts the movable valve plug to move, the second coil winding can generate a magnetic field, to detect a signal change of the second coil winding, and determine whether the first coil winding normally works; and
when the second coil winding is powered on and the second magnet yoke attracts the movable valve plug to move, the first coil winding can generate a magnetic field, to detect a signal change of the first coil winding, and determine whether the second coil winding normally works.

9. The solenoid valve according to claim 3, wherein the mounting groove is an annular groove, and the annular groove is provided with an inner wall and an outer wall; and
a tooth groove is disposed on an end face of one end that is of the inner wall and that is close to the spacer.

10. The solenoid valve according to claim 3, wherein the mounting groove is an annular groove, and the annular groove is provided with an inner wall and an outer wall; and
a tooth groove is disposed on an end face of one end that is of the outer wall and that is close to the spacer.

11. The solenoid valve according to claim 3, wherein the mounting groove is an annular groove, and the annular groove is provided with an inner wall and an outer wall; and
tooth grooves are disposed on end faces of ends that are of the inner wall and the outer wall and that are close to the spacer.

12. The solenoid valve according to claim 3, wherein the valve body comprises a first air intake vent, a first air exhaust vent, and a second air exhaust vent;
the valve plug assembly comprises a first sealing element, and the first sealing element is connected to the multistable element;
when the coil winding is powered on, the magnet yoke can attract the spacer, and the spacer drives the multistable element to move and deform, so that the spacer abuts against the magnet yoke, and the first air intake vent, the first air exhaust vent, and the second air exhaust vent communicate with each other; and
when the coil winding is powered off, the multistable element deforms and rebounds to a second steady-state position, and drives the first sealing element to seal the first air intake vent, wherein
when the coil winding is powered on, a buckling deformation distance of the multistable element is less than Y4.

13. The solenoid valve according to claim 3, wherein the valve body comprises a first air intake vent and a first air exhaust vent;
a first breather hole is disposed on the magnet yoke, and the first breather hole communicates with the first air exhaust vent;
the valve plug assembly further comprises a first sealing element and a magnetic element, the first sealing element is disposed at one end that is of the magnetic element and that is close to the first air intake vent, and the magnetic element can magnetize the spacer;
when the coil winding is powered off, the magnet yoke attracts the spacer, and the spacer drives the multistable element to move and deform to a first steady-state position, so that the spacer abuts against the magnet yoke, and the first air intake vent communicates with the first air exhaust vent through the first breather hole, to open the air path; and
when the coil winding is powered on, under an action of a magnetic field, the magnet yoke repels the spacer, and the spacer drives the first sealing element to seal the first air intake vent, to block the air path.

14. The solenoid valve according to claim 3, wherein the valve body comprises a first air intake vent and a first air exhaust vent;
a first breather hole is disposed on the magnet yoke, and the first breather hole communicates with the first air exhaust vent;
the valve plug assembly further comprises a first sealing element and a magnetic element, the first sealing element is disposed at one end that is of the magnetic element and that is close to the first air exhaust vent, and the magnetic element can magnetize the spacer;
when the coil winding is powered off, the magnet yoke attracts the spacer, the spacer drives the sealing element to seal the first breather hole, and the multistable element moves and deforms to a second steady-state position, to block the air path; and
when the coil winding is powered on, under an action of a magnetic field, the magnet yoke repels the spacer, and the spacer drives the multistable element to deform, so that the first sealing element is away from the first breather hole, and the first air intake vent communicates with the first air exhaust vent through the first breather hole, to open the air path.

15. The solenoid valve according to claim 14, wherein along a height direction of the solenoid valve, a cross-sectional area of the first air intake vent is greater than a cross-sectional area of the magnetic element.

16. A solenoid valve, wherein the solenoid valve comprises:
a valve body, wherein the valve body has an accommodating cavity;
a first magnet yoke, wherein the first magnet yoke is disposed in the accommodating cavity, and the first magnet yoke has a first mounting groove;
a first coil winding, wherein the first coil winding is disposed in the first mounting groove;
a valve plug assembly, wherein the valve plug assembly is disposed in the accommodating cavity and can move relative to the valve body; and
an elastic element, wherein the elastic element is disposed in the accommodating cavity, and two ends of the elastic element are respectively connected to the valve plug assembly and an inner wall of the valve body, wherein
under elastic force of the elastic element, the elastic element can press the valve plug assembly, so that the valve plug assembly blocks or opens an air path of the solenoid valve; and
when the first coil winding is powered on, the first magnet yoke can drive the valve plug assembly to move, so that the valve plug assembly can overcome the elastic force of the elastic element to open or block the air path of the solenoid valve.

17. The solenoid valve according to claim 16, wherein the valve plug assembly comprises a spacer and a first sealing element, and the spacer is connected to the first sealing element;
the valve body has a first air intake vent and a first air exhaust vent, and the first sealing element is disposed close to the first air intake vent;
when the first coil winding is powered off, under elastic force of the elastic element, the spacer presses the first sealing element to seal the first air intake vent, to block the air path; and
when the first coil winding is powered on, the first magnet yoke attracts the spacer, the spacer overcomes the elastic force of the elastic element to drive the first sealing element to move in a direction away from the first air intake vent, and the first air intake vent communicates with the first air exhaust vent, to open the air path.

18. The solenoid valve according to claim 17, wherein a recessed portion is disposed on the first magnet yoke, a protrusion portion corresponding to the recessed portion is disposed on the spacer, and the protrusion portion is disposed in the recessed portion; and
along a height direction of the solenoid valve, the protrusion portion can move along the recessed portion.

19. The solenoid valve according to claim 16, wherein the valve plug assembly comprises a spacer and a first sealing element, and the spacer is connected to the first sealing element;
the valve body has a first air intake vent and a first air exhaust vent, and the first sealing element is disposed close to the first air exhaust vent;
a first breather hole is disposed on the first magnet yoke, and the first breather hole communicates with the first air exhaust vent;
when the first coil winding is powered off, the spacer is pressed under elastic force of the elastic element, so that the first sealing element is away from the first breather hole, and the first air intake vent communicates with the first air exhaust vent through the first breather hole, to open the air path; and
when the first coil winding is powered on, the first magnet yoke attracts the spacer, and the spacer overcomes the elastic force of the elastic element to drive the first sealing element to seal the first breather hole, to block the air path.

20. A device, configured to measure blood pressure, wherein the device comprises:
a device body;
an airbag, mounted on the device body, wherein the airbag is configured to be worn on a measured part;
an air pump, mounted on the device body, wherein the air pump is configured to provide fluid to the airbag;
a pressure sensor, mounted on the device body, wherein the pressure sensor is configured to detect pressure of the airbag;
a solenoid valve, wherein the solenoid valve is mounted on the device body, and the solenoid valve is the solenoid valve according to any one of claims 1 to 19; and
a detecting element, wherein the detecting element is configured to detect whether the solenoid valve normally works.
